# EUROPÄISCHE PATENTANMELDUNG

(11) **EP 1 710 232 A1**
(43) Veröffentlichungstag der Anmeldung: **11.10.2006**
(21) Anmeldenummer: 06113758.4
(22) Anmeldetag: 25.10.1996
(51) Int. Cl.: C07C 323/58, C07C 319/28

(54) **Verfahren zur Freisetzung von D,L-Methionin**

(30) Priorität: 18.12.1995 DE 19547236
(62) Teilanmeldung aus: 02011911.1
(71) Anmelder: Degussa AG, 40474 Düsseldorf (DE)
(72) Erfinder: Geiger, Friedhelm, Erlensee 63526 (DE); Theissen, Ferdinand, Bornheim 53332 (DE); Tanner, Herbert, Hanau-Grossauheim 63457 (DE); Mannsfeld, Sven Peter, Lauf/Pegnitz 91207 (DE); Körfer, Martin, Kalmthout-Heide 2920 (BE); Huthmacher, Klaus, Gelnhausen 63571 (DE); Hentschel, Klaus, Rodenbach 63517 (DE); Hasselbach, Hans-Joachim, Gelnhausen 63571 (DE); Halsberghe, Baudouin, 2040 Antwerpen 4 (BE); Willigerodt, Klaus, Bonn 53225 (DE); Vanrobaeys, Jose, Kalmthout 2920 (BE)

(57) **Zusammenfassung**

Die Erfindung betrifft ein Verfahren zur Freisetzung von DL-Methionin aus Alkalimethioninat mit Kohlendioxid in Gegenwart eines Entschäumers.

## Beschreibung

Die Erfindung betrifft ein Verfahren zur Freisetzung von D,L-Methionin aus einem Alkalimethioninat in Gegenwart eines Entschäumers.

Die Synthese lässt sich durch folgende Reaktionsgleichungen veranschaulichen: 5-(2-Methylmercapto)-hydantoinbildung: 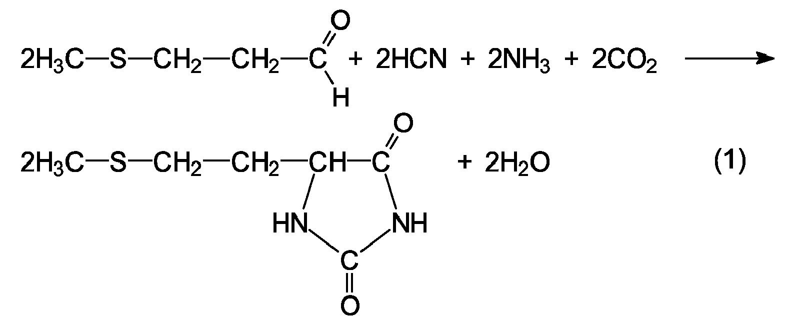
Salzbildung des D,L-Methionins: 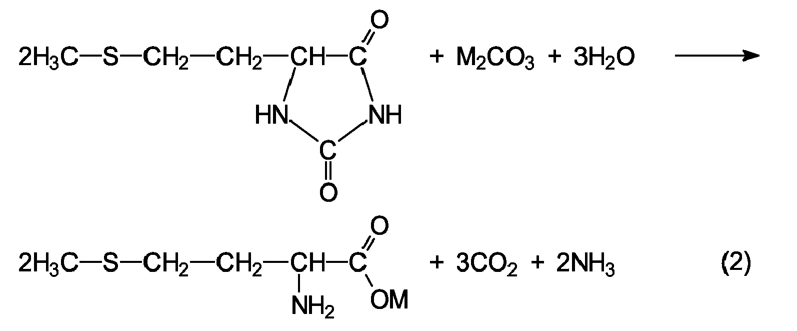
D,L-Methionin-Freisetzung:

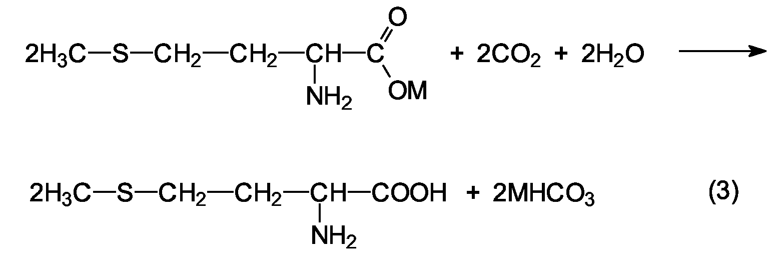

M steht für Alkali, insbesondere Kalium.

Die Verfahrensstufen 5-(2-Methylmercaptoethyl)-hydantoin-Bildung, Methioninat-Bildung sowie Methionin-Freisetzung können für sich vorteilhaft kontinuierlich betrieben werden und werden zweckmäßigerweise hintereinander verschaltet in einen insbesondere gesamtkontinuierlich verlaufenden Prozeß integriert.

Besonders vorteilhaft werden die Komponenten Ammoniak und Kohlendioxid entsprechend den Prozeßmöglichkeiten recycliert, d. h. in die zuvor ablaufenden Verfahrensstufen wieder eingesetzt. Insbesondere bei der Verwendung von Kalium werden möglichst die gesamten Alkali enthaltenden Agentien in den Prozeß zurückgeführt.

Die 5-(2-Methylmercapto)-Hydantoin-Bildung ist grundsätzlich bekannt. Generell wird dabei entweder von den oben unter 1) beschriebenen Komponenten ausgegangen oder von solchen Komponenten, aus denen diese Komponenten herstellbar sind. Diese sind insbesondere Alkali oder Ammoniumsalze bei den Komponenten Cyanwasserstoff, Ammoniak und Kohlendioxid bzw. Acrolein und Methylmercaptan bei der Komponente 3-Methylmercaptopropionaldehyd.

Ein nach den bekannten Verfahren hergestelltes 5-(2-Methylmercaptoethyl)-hydantoin enthält in erheblichem Umfang Verunreinigungen durch 5-(2-Methylmercaptoethyl)-hydantoinsäure, 5-(2-Methylmercaptoethyl)-hydantoinsäureamid, Methioninamid, Methioninnitril sowie Methylmercaptopropionaldehydcyanhydrin, Iminonitril und Polymere. Während die drei zuerst genannten Verbindungen bei der alkalischen Hydrolyse wie das Hydantoin in Methionin überführt werden, gelangen die übrigen Verbindungen bzw. deren Verseifungsprodukte in die Verseifungslösung und in das später zu isolierende Methionin, wo sie sich nur sehr schwierig abtrennen lassen. Dies ist um so mehr der Fall, wenn das Methionin aus dem Hydantoin hergestellt und mittels Kohlendioxid aus dem Umsetzungsgemisch abgeschieden und die Mutterlauge im Kreislauf geführt wird. Das erhaltene Methionin ist verfärbt und zeigt eine schlechte Lagerbeständigkeit.

Die alkalische Hydrolyse von 5-(2-Methylmercaptoethyl)-hydantoin ist seit langem bekannt. So beschreiben die US-A 2,527,366 und US-A 2,557,913 die Hydrolyse von 5-(2-Methylmercaptoethyl)-hydantoin in einer wäßrigen Bariumhydroxidlösung unter Druck und erhöhter Temperatur. Diese Verfahren erfordern aber erhebliche Mengen an teurem Bariumhydroxid, außerdem muß das Barium als Neutralsalz wieder abgetrennt werden.

Aus der US-A 2,557,920 ist bekannt, daß α-Aminosäuren durch Verseifung von Hydantoinen unter Verwendung von Natriumhydroxid entstehen. Bei diesen Verfahren werden aber mindestens 3 Mol Natriumhydroxid je Mol Hydantoin benötigt. Analog verhält es sich beim Einsatz von Kaliumhydroxid.

Ferner ist aus der US-A 4,272,631 bekannt, daß ein Gemisch aus Alkali- und Erdalkalihydroxid zur Verseifung von 5-(2-Methylmercaptoethyl)-hydantoin verwendet werden kann. Allerdings müssen bei diesen Verfahren die Erdalkaliionen bei der Freisetzung von Methionin erst abgetrennt werden, so daß nur Ausbeuten von max. 80,5 % erreicht werden.

In der US-A 4,259,925 wird die Hydrolyse von 5-(2-Methylmercaptoethyl)-hydantoin unter Druck bei 105 bis 230 °C in einem Medium, das ein Metallhydroxid und einen Alkohol mit einem Siedepunkt von 125 bis 130 °C enthält, durchgeführt. Nachteilig ist, daß der hochsiedende Alkohol wieder zurückgewonnen werden muß. Außerdem beträgt die Ausbeute nur 65 %.

In der DE-PS 19 06 405 wird die Hydrolyse von 5-(2-Methylmercaptoethyl)-hydantoin unter Verwendung einer wässrigen Lösung von Alkalicarbonat und/oder Alkalihydrogencarbonat beschrieben. Während der Hydrolyse werden Ammoniak und Kohlendioxid laufend entfernt. Von den Alkalicarbonaten wird Kaliumcarbonat bevorzugt; es wird ein Mol-Verhältnis Hydantoin zu Alkali von 1 : 1 bis 1 : 5 angewendet. Die Hydrolyse wird unter Druck bei 120 bis 220 °C durchgeführt. Die kontinuierliche Druckapparatur besteht aus drei aufwendig hintereinander geschalteten Umlaufverdampfern. Die Alkalimethioninatlösung wird zur Freisetzung von D,L-Methionin mit Kohlendioxid verwendet; die Mutterlauge aus der Abtrennung des auskristallisierten Methionins wird im Kreislauf ggf. mit Ausschleusung von 1 - 2 % wieder zur Hydrolyse des Hydantoins eingesetzt.

Die DE-AS 15 18 339 beschreibt ein Verfahren, bei dem bei der Hydrolyse entstandene gasförmige Reaktionsprodukte (Ammoniak und Kohlendioxid) aus der Reaktion entfernt werden, um das Reaktionsgleichgewicht in Richtung der Aminosäure zu verschieben, wodurch die Ausbeute gesteigert wird. Um dies zu erreichen, ist jedoch eine komplexe apparative Anordnung zur Druckregelung der Gasströme erforderlich.

Die japanische Patentschrift 49/116 008 beschreibt ein Verfahren, bei dem die Hydrolyse von 5-(2-Methylmercaptoethyl)-hydantoin in Gegenwart von Vanadinsäuren, Molybdänsäuren, Wolframsäuren oder deren Derivate durchgeführt wird. Die Ausbeuten betragen ca. 70 %. Schwierig ist die Abtrennung des Katalysators. Zur Herstellung einer hochkonzentrierten Methionin-haltigen Lösung muß Alkali, z. B. eine Kaliumverbindung, zugesetzt werden.

Aus der japanischen Patentanmeldung 75/106 901 (C. A. 84, 44666k (1976)) ist bekannt, daß Methionin durch Hydrolyse von 5-(2-Methylmercaptoethyl)-hydantoin in Gegenwart von etwa 1,2 eq Natriumhydroxid und von etwa 9 eq Ammoniak bei 180 °C hergestellt wird. Die bei dieser Arbeitsweise intermediär entstehende Natriummethioninat-Lösung enthält jedoch zwangsläufig neben dem Natriummethioninat auch Natriumcarbonat, das bei dieser Reaktionsführung ausfällt und deshalb insbesondere bei einem kontinuierlichen Prozeß störend ist. Analoges gilt bei der Verwendung von Kaliumhydroxid sowie für die Verfahrensweise gemäß

DE-PS 19 06 405.

Die DE 26 14 411 A beschreibt die Hydrolyse von 5-(2-Methylmercaptoethyl)-hydantoin mit Wasser in Gegenwart von Imidazol bei 160 °C. Die Ausbeuten sind niedrig und auch hier muß eine Alkaliverbindung zur Erzeugung einer hohen Lösungskonzentration zugegeben werden.

Die japanischen Patentanmeldungen JP 03/95145 und JP 03/95146 beschreiben die Hydrolyse von Hydantoinen mit Wasser bei erhöhter Temperatur und erhöhtem Druck in Gegenwart von Metalloxiden oder -oxidgemischen, wie z. B. ZrO₂, TiO₂, Nb₂O₅ oder TiO₂-Nb₂O₅; die Ausbeuten liegen allerdings nur bei 65 bis 66 %. Diese Lösungen müssen mit einer Alkaliverbindung neutralisiert werden.

All diese Verfahren bringen entweder geringe Ausbeuten oder haben den Nachteil, daß Methionin oder Salze wie Carbonate während des Prozesses ausfallen, wodurch weitere Verfahrensschritte verursacht werden und insbesondere großtechnische Verfahren, ganz besonders kontinuierliche Verfahren, kaum möglich sind.

Die Freisetzung des Methionins aus dem Alkalisalz ist allgemein bekannt. Nach dem Prinzip, starke Säuren setzen schwächere Säuren aus ihren Salzen frei, wird z. B. mit Salzsäure, Schwefelsäure, Phosphorsäure oder einem stark sauren Ionenaustauscher freies D,L-Methionin ausgefällt (DE 21 40 506 C; DE 21 22 491 C; DE 29 12 066 A; BE 877 200, US-A 3 433 832, FR 1 532 723). Allerdings muß dann noch das als Nebenprodukt anfallende Alkalisalz abgetrennt werden. Da die eingesetzte Säure im allgemeinen nicht mehr zurückgewonnen wird, ist diese Vorgehensweise für ein kontinuierliches, wirtschaftliches und umweltschonendes Herstellverfahren ungeeignet.

Zweckmäßigerweise wurde deshalb - wie beispielsweise in den Patentschriften US-A 2 557 913, DE-PS 19 06 405 und JP 42/44056 beschrieben - D,L-Methionin aus den Hydrolyselösungen des 5-(2-Methylmercaptoethyl)-hydantoins mit Kohlendioxid in wässriger Lösung ausgefällt. Bei dieser Methode fällt i. d. R. das D,L-Methionin in Form von dünnen Plättchen oder schuppenförmig an. Dies bedingt eine schlechte Filtrierbarkeit des Produkts und behindert ein Auswaschen des Kristallkuchens; außerdem fällt ein D,L-Methionin mit schlechtem Fließverhalten und Neigung zur Verklumpung an. Um diesen Nachteilen entgegenzuwirken, werden gemäß der japanischen Patentschrift JP 42/44056 Zusätze wie Kasein oder wasserlösliche, hochmolekulare Cellulosederivate bei der Methioninfällung mit Kohlendioxid zugesetzt.

Aufgabe der vorliegenden Erfindung ist ein Verfahren zur Herstellung von Methionin aus einem Alkalimethioninat, bei dem möglichst wenig Nebenprodukte entstehen sollen und dessen einzelne Komponenten sich gut abtrennen lassen sollen, insbesondere soll dieses bei der Ausfällung eines Produktes dieses gut filtrierbar sein. Einzelne Komponenten sollen weitgehend recyclierbar bzw. regenerierbar sein. Außerdem soll das erhaltene Methionin nur geringe Verfärbungen haben und gut lagerbeständig sein. Das Verfahren soll insbesondere im großtechnischen Maßstab sowie kontinuierlich möglich sein.

Erfindungsgemäß wird dies gelöst ausgehend von einem Verfahren zur Herstellung eines Alkalisalzes des Methionins beginnend mit der Umsetzung der Komponenten 3-Methylmercaptopropionaldehyd, Cyanwasserstoff, Ammoniak und Kohlendioxid oder von solchen Komponenten, aus denen die vorgenannten Komponenten herstellbar sind, ggf. in Gegenwart von Wasser, zum 5-(2-Methylmercaptoethyl)-hydantoin und weiterer Umsetzung desselben zum Methionin, wobei die Umsetzung der Komponenten über mindestens eine Vormischung derart eingeleitet wird, daß man ein erstes Gemisch bildet, enthaltend zumindest den überwiegenden Teil (mind. 5/10) des 3-Methylmercaptopropionaldehyds und mindestens 1/10 der Komponente Cyanwasserstoff oder entsprechende Mengen solcher Komponenten, aus denen diese Komponenten herstellbar sind, und weniger als 5/10 einer der Komponenten Ammoniak, Kohlendioxid oder der Komponenten, aus denen Ammoniak oder Kohlendioxid herstellbar sind, und daß man dieses erste Gemisch mit der oder den weiteren Komponenten zur reaktiven Umsetzung zum 5-(2-Methylmercaptoethyl)-hydantoin vereinigt, wobei diese letztere(n) Komponente(n) in einem oder mehreren weiteren Gemischen vorgemischt sein können.

Unter Komponenten, aus denen die vorgenannten Komponenten herstellbar sind, fallen z. B. Salze von Cyanwasserstoff, Ammoniak und Kohlendioxid, wie z. B. Natrium oder Kaliumcyanid, Ammoniumcarbonat oder -bicarbonat, Natrium oder Kaliumcarbonat oder -bicarbonat und natürlich insbesondere deren Lösungen in Wasser. Entsprechende Komponenten für 3-Methylmercaptopropionaldehyd sind Acrolein und Methylmercaptan. Grundsätzlich ist es bevorzugt, bei dieser Reaktion eventuell eingesetzte Metallsalze möglichst im Unterschuß einzusetzen, d. h., daß die Aldehydkomponente und/oder die Cyanidkomponente im stöchiometrischen Überschuß zu einer eventuell eingesetzten Metallionenkomponente ist. Besonders bevorzugt wird hier möglichst kein Metallsalz, wie z. B. Natrium oder Kaliumcyanid, eingesetzt sondern die oben genannten Verbindungen. Insbesondere bei einem kontinuierlichen Prozeß können aber zusätzlich andere Metallsalze, z. B. Katalysatoren o. ä., zugegen sein. Deren Dosierung fällt nicht unter die oben angegebene bevorzugte Metallionenbeschränkung hinsichtlich der Reaktionskomponenten.

Hier und im folgenden wird unter den angegebenen Komponenten auch deren unter den gegebenen Einsatzbedingungen veränderte Einsatzform verstanden. So liegt z. B. beim Einsatz von Ammoniak und Kohlendioxid in Wasser ein Teil dieser Komponenten als Ammonium(hydrogen)carbonat vor.

Nach diesem Verfahren wird das 5-(2-Methylmercaptoethyl)-hydantoin farblos mit praktisch quantitativer Ausbeute und so weitgehend frei von Verunreinigungen gewonnen, daß daraus ein Methioninsalz in einem kontinuierlichen Prozeß unter Mutterlaugenrecyclierung erhalten werden kann, das sich durch außergewöhnliche Lagerstabilität bezüglich Verfärbung und Verklumpung auszeichnet.

Vorzugsweise enthält das erste Gemisch mindestens 5/10 der Cyanwasserstoff-Komponente, bevorzugt 9/10 und insbesondere mindestens 99/100 oder entsprechende Anteile der Komponente, aus der Cyanwasserstoff herstellbar ist. Die obigen Bruchangaben (z. B. 5/10) sowie die folgenden beziehen sich immer auf die jeweils angegebene Komponente selbst (nicht auf die Stöchiometrie des Verfahrens). Es ist also unabhängig von der Stöchiometrie die gesamte eingesetzte Menge einer Komponente 1/1.

Insbesondere ist es bei dem Verfahren vorteilhaft, wenn Ammoniak und Kohlendioxid oder solche Komponenten, aus denen Ammoniak oder Kohlendioxid herstellbar sind, jeweils zu weniger als 5/10 ihrer Einsatzmengen, bevorzugt zu höchstens 1/10 und insbesondere zu höchstens 1/100, im ersten Gemisch eingesetzt werden.

Vorteilhaft ist es auch, wenn das ggf. der Reaktion zugesetzte Wasser im ersten Gemisch zu höchstens 5/10, bevorzugt höchstens 1/10 und insbesondere höchstens 1/100 enthalten ist.

Vorteilhaft ist es auch, wenn beim Beginn der Umsetzung alle Komponenten in ihrer vorgesehenen Einsatzmenge vollständig vorhanden sind, d. h., daß keine Komponente nachdosiert wird. Vorteilhaft ist es außerdem, wenn alle Komponenten in insgesamt zwei Vormischungen vereinigt werden, die dann zur Umsetzung miteinander vermischt werden.

Bei allen Umsetzungen ist es besonders günstig, wenn die einzelnen Komponenten bzw. Vormischungen schnell und möglichst innig miteinander vermischt werden.

Bevorzugt enthält das Reaktionsgemisch keine organischen Lösungsmittel, wenn organische Lösungsmittel eingesetzt werden, dann vorteilhaft zu weniger als 20 Gew.-Teile, insbesondere zu weniger als 10 Gew.-Teile, bezogen auf 100 Gew.-% Wasser.

Besonders vorteilhaft ist es, wenn die Vormischung(en) und ggf. einzelne Komponenten (es können eine oder mehrere Vormischungen miteinander zur Reaktion gebracht werden, wobei im Falle von zwei Vormischungen ggf. alle einzelnen Komponenten in diesen untergebracht sein können) in ein bereits erhaltenes Umsetzungsgemisch, enthaltend 5-(2-Methylmercaptoethyl)-hydantoin eingebracht werden. Hierbei ist es besonders vorteilhaft, wenn alle Vormischung(en) und ggf. einzelne Komponenten entweder benachbart oder mit einer zeitlichen (bei chargenweiser Herstellung) oder strömungsbedingten (bei kontinuierlicher Herstellung) Versetzung um höchstens 30 s in das Umsetzungsgemisch eingeleitet werden.

Die Reaktion wird bei der oben beschriebenen Verfahrensweise vorzugsweise bei einer Temperatur über 80 °C ausgeführt; außerdem wird die Reaktion vorzugsweise bei Überdruck, besonders bevorzugt bei einem Druck größer als dem sich einstellenden Gleichgewichtsdruck (Reaktionsdruck), insbesondere bei mind. 3 bar und besonders vorteilhaft bei über 10 bar, durchgeführt.

Das oben beschriebene Verfahren mit seinen Varianten eignet sich insbesondere zur kontinuierlichen Durchführung.

Bei dem Verfahren zur Freisetzung von Methionin aus einem Alkalisalz des Methionins sind insbesondere zwei der oben beschriebenen Verfahrensmaßnahmen für sich allein schon besonders vorteilhaft. Die Beschreibung enthält ein Verfahren zur kontinuierlichen Herstellung eines Alkalisalzes des Methionins betrifft, mit der Umsetzung der Komponenten 3-Methylmercaptopropionaldehyd, Cyanwasserstoff, Ammoniak und Kohlendioxid oder von solchen Komponenten, aus denen die vorgenannten Komponenten herstellbar sind, ggf. in Gegenwart von Wasser, zum 5-(2-Methylmercaptoethyl)-hydantoin und weiterer Umsetzung desselben zu einem Alkalisalz des Methionins, wobei die Komponenten in ein Umsetzungsgemisch eingebracht werden, das aus den vorgenannten Komponenten gebildet ist und bereits mindestens 1/10 der theoretisch bildbaren Menge des 5-(2-Methylmercaptoethyl)-hydantoins enthält, und wobei die Umsetzung bei Überdruck, bevorzugt bei einem Druck von mindestens 3 bar, durchgeführt wird.

Hier ist es besonders vorteilhaft, wenn bei einem Druck von mind. 7 bar und insbesondere bei einem Druck von mind. 10 bar gearbeitet wird. Außerdem ist es besonders vorteilhaft, wenn die Komponenten unmittelbar oder mit einer strömungsbedingten Vorreaktionszeit von höchstens 30 s in das Umsetzungsgemisch eingeleitet werden, d. h., daß die einzelnen Komponenten höchstens 30 s in reaktivem Kontakt stehen, bevor sie in das Umsetzungsgemisch eingebracht werden.

Außerdem sind auch bei dieser Verfahrensvariante die oben beschriebenen einzelne vorteilhafte Maßnahmen durchführbar.

Die bis jetzt beschriebenen Verfahren werden besonders bevorzugt als ein Verfahren durchgeführt, wobei im nachfolgenden eine entsprechende summarische Darstellung dieses Verfahrens wiedergegeben wird. Die hierbei gegebenen weiteren Spezifikationen, z. B. hinsichtlich der Stöchiometrie, der Temperaturen, etc., gelten jeweils für sich allein auch vorteilhaft für die oben allgemein beschriebenen Verfahren.

5-(2-Methylmercaptoethyl)-hydantoin wird besonders vorteilhaft hergestellt, indem eine Lösung von Cyanwasserstoff in 3-Methylmercaptopropionaldehyd und eine Lösung von Ammoniak und Kohlendioxid in Wasser bereitet wird und diese Lösungen schnell und innig miteinander vermischt und zur Umsetzung gebracht werden. Ammoniak und Kohlendioxid können aus der Hydrolysestufe dieses Hydantoin zurückgeführt werden. Die Lösung von Cyanwasserstoff in 3-Methylmercaptopropionaldehyd wird zweckmäßigerweise so eingestellt, daß sie aus equimolaren Anteilen Cyanwasserstoff und 3-Methylmercaptopropionaldehyd besteht, oder aber überschüssige Anteile an Cyanwasserstoff enthält. Im allgemeinen ist es vorteilhaft, den Anteil Cyanwasserstoff in der Lösung nicht größer als 1.1 Mol je Mol 3-Methylmercaptopropionaldehyd zu wählen; vorzugsweise enthält die Lösung 1,005 bis 1,05 Mol Cyanwasserstoff je Mol 3-Methylmercaptopropionaldehyd.

Bei der Lösung von Ammoniak und Kohlendioxid in Wasser kann es sich um eine gesättigte oder verdünnte Lösung handeln; vorteilhaft ist der Gehalt an Ammoniak nicht unter etwa 5 Gew.-%. In der Lösung kann Ammoniumhydrogencarbonat, Ammoniumcarbonat, Carbaminsäure, Ammoniumcarbamidat, Cyansäure oder ein Gemisch aus diesen Komponenten vorliegen. Das Molverhältnis Ammoniak zu Kohlendioxid beträgt zweckmäßigerweise etwa 1,2 bis 4,0 Mol, vorzugsweise 1,6 bis 1,8 Mol Ammoniak je Mol Kohlendioxid. Die Lösung des Cyanwasserstoffs in 3-Methylmercaptopropionaldehyd wird mit der Lösung des Ammoniaks und Kohlendioxids in Wasser so vermischt, daß in der Mischung zweckmäßigerweise ein Molverhältnis Ammoniak zu 3-Methylmercaptopropionaldehyd von etwa 1,2 bis 6 zu 1,0, vorzugsweise 2,0 bis 4,0 zu 1,0, insbesondere 2,5 bis 3,0 zu 1,0, vorliegt.
Die Umsetzung wird bei Umgebungstemperatur oder darüber, zweckmäßigerweise bei Temperaturen über 60 °C, vorteilhaft etwa zwischen 80 °C und 140 °C, ausgeführt. Vorzugsweise werden Temperaturen zwischen 80 und 130 °C, insbesondere zwischen 90 und 120 °C, gewählt. Wenngleich die Umsetzung bei beliebigem Druck ablaufen kann, ist es zweckmäßig, bei erhöhtem Druck zu arbeiten; vorteilhaft erweisen sich Drücke bis 20 bar, insbesondere Drücke, die 2 bis 3 bar über dem Gleichgewichtsdruck des Umsetzungsgemisches liegen. Die Umsetzungszeit richtet sich nach den Umsetzungsbedingungen, insbesondere nach der Temperatur und nach den Mengenverhältnissen.

Bei der bevorzugten Arbeitsweise ist es besonders vorteilhaft, die Lösung des Cyanwasserstoffs in 3-Methylmercaptopropionaldehyd und die Lösung des Ammoniaks und Kohlendioxids im Wasser in ein Umsetzungsgemisch dieser Substanzen, d. h. in ein zuvor bei der Umsetzung der Lösungen entstandenes Gemisch, in dem ganz oder teilweise die Umsetzung vom Hydantoin erfolgt ist, einzutragen und in diesem Gemisch die Umsetzung auszuführen.

Besonders vorteilhaft ist es, eine kontinuierliche Arbeitsweise zu wählen, hierzu das Umsetzungsgemisch im Kreislauf zu führen, in diesem Kreislauf an zwei benachbarten Stellen stetig die Lösungen von Cyanwasserstoff in 3-Methylmercaptopropionaldehyd und vom Ammoniak und Kohlendioxid in Wasser einzuspeisen und an einer anderen Stelle stetig aus dem Kreislauf einen entsprechenden Anteil des Umsetzungsgemisches abzuziehen.

Obwohl das Mischungsverhältnis zwischen den Lösungen, die in den Kreislauf eingespeist werden, und dem Umsetzungsgemisch, das im Kreislauf geführt wird, beliebig sein kann, ist es zweckmäßig, dieses Mischungsverhältnis so zu wählen, daß je Volumenteil der Lösungen mehrere Volumenteile des Umsetzungsgemisches, ggf. 1000 oder mehr, bevorzugt 5 bis 100, insbesondere 10 bis 25 Volumenteile des Umsetzungsgemisches, vorliegen. Von entscheidendem Vorteil ist es, die in den Kreislauf eingespeisten Lösungen mit dem im Kreislauf geführten Umsetzungsgemisch schnell und innig zu vermischen; dieses kann ggf. über eine Mischdüse, einen statischen Mischer, durch eine hohe Kreislaufzirkulation oder eine Kombination aller Maßnahmen bewerkstelligt werden. In den aus dem Kreislauf abgezogenen Anteilen des Umsetzungsgemisches ist die Umsetzung ggf. nicht vollständig abgelaufen, so daß es vorteilhaft sein kann, diese Anteile noch einige Zeit in einem Nachreaktor ausreagieren zu lassen. Falls weniger als 5 Volumenteile des Umsetzungsgemisches auf ein Volumenteil der Lösungen vorliegen, besteht die Gefahr der Polymerisation der Ausgangskomponenten. Das Gemisch färbt sich dunkel, es kommt zu Ausfällungen und damit zu Ausbeuteverlusten und/oder technischen Störungen.

Gemäß der vorliegenden Beschreibung wird das wie oben beschrieben hergestellte, jedoch auch auf andere Art und Weise hergestelltes, 5-(2-Methylmercaptoethyl)-hydantoin zu einem Alkalisalz des Methionins und weiter zum Methionin umgesetzt. Das Alkalisalz des Methionins wird durch Hydrolyse von 5-(2-Methylmercaptoethyl)-hydantoin in Gegenwart einer wässrigen Lösung enthaltend Alkali und Kohlendioxid hergestellt, wobei die Hydrolyse zumindest zu Beginn in Gegenwart von mind. 0,1 eq, insbesondere bis 7 eq, Ammoniak je Equivalent 5-(2-Methylmercaptoethyl)-hydantoin durchgeführt wird. Ein Alkalisalz des Methionins kann ebenfalls durch Hydrolyse von 5-(2-Methylmercaptoethyl)-hydantoin in Gegenwart einer wässrigen Lösung enthaltend Alkali und Kohlendioxid hergestellt und erfindungsgemäß weiter zum Methionin umgesetzt werden, wobei die Hydrolyse in Gegenwart von metallischem Zirkonium oder einer Zirkonium-Legierung, enthaltend mind. 10 Gew.-% Zirkonium, durchgeführt wird.

Besonders vorteilhaft ist es, wenn beide Verfahren kombiniert werden.

Es hat sich gezeigt, daß es besonders vorteilhaft ist, wenn die Hydrolyse von Anfang an in Gegenwart von Alkali und Kohlendioxid, d. h., daß insbesondere eine Mischung von Alkaliverbindungen vorliegt, insbesondere Alkalihydrogencarbonat, Alkalicarbonat, Alkalihydroxid, wobei Alkali insbesondere für Kalium und Natrium steht, durchgeführt wird. Die Menge von Alkali und Kohlendioxid ist dabei zweckmäßig wenigstens die auf das Hydantoin bezogene stöchiometrische Menge. Diese kann nach oben deutlich überschritten werden. Ein Molverhältnis mit einem Überschuß von etwa 3 : 1 bezogen auf das Hydantoin ist besonders vorteilig; grundsätzlich ist davon auszugehen, daß ein noch größerer Überschuß noch günstiger ist. Für die Praxis jedoch sind Verhältnisse von etwa 1,5 : 1 - 2 : 1 besonders bevorzugt. Erfindungsgemäß wird dabei noch zusätzlich etwas Ammoniak zugegeben, der entsprechend ebenfalls teils auch in Form von Ammoniumverbindungen vorliegt. Besonders vorteilhaft ist es hierbei, wenn zu Beginn der Hydrolyse je Mol 5-(2-Methylmercaptoethyl)-hydantoin max. 7 Mol Ammoniak (incl. Ammoniumverbindungen) vorliegt. Hierdurch wird erreicht, daß die Hydrolyse praktisch ohne Nebenproduktbildung und in guten Ausbeuten abläuft und andererseits nicht oder nur wenig Alkalicarbonat ausfällt. Besonders vorteilhaft ist hierbei, wenn während der Hydrolyse Ammoniak und/oder Kohlendioxid, ggf. zusammen mit Wasser, aus dem Reaktionssystem ausgeschleust werden. Hierdurch lassen sich die Reaktionsbedingungen besonders günstig steuern, so daß weiterhin kein Alkalicarbonat ausfällt und die Reaktion vollständig verläuft. Besonders vorteilhaft ist es, wenn die Hydrolyseapparatur selbst Zirkoniumeinbauten enthält (aus Zirkonium oder aus einer entsprechenden Zirkoniumlegierung ist). Es wurde festgestellt, daß das Zirkonium einen besonders günstigen vermutlich katalytischen Effekt auf die Hydrolyse ausübt. Als günstiger Nebeneffekt ergibt sich hierbei, daß diese Apparate gut widerstandsfähig und somit langlebig sind, so daß der Einsatz von Zirkoniumapparaturen keine apparatebedingten Nachteile gegenüber anderen Apparaturen bringt.

Die Hydrolyseverfahren werden günstigerweise bei einer Temperatur von 120 bis 250 °C und entsprechend einem Druck von 5 bis 30 bar durchgeführt. In diesem Bereich ergeben sich sehr gute Umsetzungen und geringe Nebenproduktbildung. Vorteilhaft ist auch, wenn die Alkalikomponente zumindest equimolar bezüglich des 5-(2-Methylmercaptoethyl)-hydantoins eingesetzt wird. Man erhält dann neben der vollständigen Hydrolyse auch praktisch quantitativ das entsprechende Alkalisalz des Methionins. Bevorzugt enthält die Hydrolyselösung zu Beginn auch bereits Methionin bzw. dessen Salz, auch dies hat einen günstigen, vermutlich autokatalytischen Effekt auf die Hydrolyse.

Bei dieser Verfahrensweise kann vorteilhaft im Laufe bzw. nach der Hydrolyse praktisch das gesamte Ammoniak und das gesamte Kohlendioxid aus der Hydrolyselösung abgezogen werden, so daß das Hydrolysat im wesentlichen frei von Ammoniak und Kohlendioxid entnommen werden kann.

Auch hier ist es besonders vorteilhaft, das Verfahren kontinuierlich durchzuführen. Ganz besonders vorteilhaft ist hierbei, daß die bis jetzt beschriebenen Verfahren zusammengeschaltet werden können, insbesondere als gesamtkontinuierlicher Prozeß, wobei Kohlendioxid und Ammoniak recycliert werden können.

Im folgenden wird die Hydantoin-Hydrolyse näher beschrieben, wobei einzelne nähere Spezifikationen grundsätzlich für sich allein auch für die oben gegebene allgemeine Verfahrensweise gelten. Die im folgenden wiedergegebene Verfahrensweise wird für entsprechende Kaliumverbindungen beschrieben, da es sich hierbei um die am meisten bevorzugte Ausführungsform handelt.

Eine Kaliummethioninat-Lösung wird durch Hydrolyse von 5-(2-Methylmercaptoethyl)-hydantoin in Gegenwart von Kaliumhydroxid, Kaliumcarbonat und/oder Kaliumhydrogencarbonat oder eines Gemisches davon und bei Anwesenheit von überschüssigem Ammoniak, Kohlendioxid, Kohlensäure, Cyansäure oder eines Gemisches davon in Wasser bei einer Temperatur von 120 bis 250 °C und einem Druck von 5 bis 30 bar erhalten. Vorteilhaft erfolgt die Hydrolyse des Hydantoins bezogen auf das Hydantoin in Gegenwart von 1 bis 15 eq einer oder mehrerer Kaliumverbindungen (z. B. KOH, KHCO₃, K₂CO₃, Kaliummethioninat). Während bzw. nach der Hydrolyse ist es außerdem vorteilhaft, wenn entstehendes oder noch vorhandenes Ammoniak und/oder Kohlendioxid ganz oder teilweise aus dem Reaktionssystem abgetrennt wird. Grundsätzlich kann jedes 5-(2-Methylmercaptoethyl)-hydantoin eingesetzt werden, günstigerweise wird ein Hydantoin eingesetzt, das wie oben beschrieben erhältlich ist.

Vorteilhaft ist das Ammoniak zu Beginn der Hydrolyse ein Mol-Verhältnis zu Kohlendioxid von 1,1 bis 8,0. Günstig ist auch ein Mol-Verhältnis des Ammoniaks zum Hydantoin von 0,2 bis 5. Bei der beschriebenen Verfahrensweise ist es möglich, das Ammoniak und das Kohlendioxid unmittelbar aus dem oben beschriebenen Verfahren, der Hydantoin-Herstellung, zu übernehmen, so daß das Hydantoin unmittelbar aus der Hydantoin-Herstellung zusammen mit dem noch verbliebenen Ammoniak und Kohlendioxid in die Hydrolyse eingebracht werden kann, wobei hier dann noch eventuell gewünschte andere Ammoniak- und/oder Kohlendioxidkonzentrationen eingestellt werden können.

Die Hydrolyse des 5-(2-Methylmercaptoethyl)-hydantoins findet bei Temperaturen von 120 - 250 °C, vorzugsweise von 150 - 200 °C, insbesondere von 160 - 180 °C, statt; der Druck während der Reaktion soll 5 - 30 bar, vorzugsweise 5 - 10 bar, insbesondere 7 - 9 bar, betragen.

Das Verfahren wird vorteilhaft in einer mit Dampf beheizten Kolonne mit Einbauten durchgeführt, bei der die Innenwand und die Einbauten aus Zirkonium oder einer mind. 10 Gew.-% Zirkonium enthaltenden Zirkoniumlegierung aufgebaut sind. Die 5-(2-Methylmercaptoethyl)-hydantoin-Lösung wird vorteilhaft am Kopf der Kolonne in der Geschwindigkeit kontinuierlich zugegeben, daß das Hydrolyseprodukt, Kaliummethioninatlösung, am Boden entsprechend abgezogen werden kann; d. h., die Hydrolyse am Boden der Kolonne quantitativ abgelaufen ist. Die gasförmigen Bestandteile, Wasserdampf, Ammoniak und Kohlendioxid, werden günstigerweise am Kopf der Kolonne ausgeschleust und können vorteilhaft zur Wiederherstellung der wässrigen Ammoniak/Kohlendioxid-Lösung zur Herstellung des 5-(2-Methylmercaptoethyl)-hydantoins eingesetzt werden.

Zur Hydrolyse des 5-(2-Methylmercaptoethyl)-hydantoins wird eine wäßrige Lösung aus Kaliumhydroxid, Kaliumcarbonat und/oder Kaliumhydrogencarbonat mit einem Kaliumionengehalt von vorteilhaft 100 - 200 g, vorzugsweise 140 - 160 g, Kalium pro Liter Hydrolyselösung eingesetzt, wobei die aus Zirkonium bestehenden Apparatewandungen und Einbauten einen günstigen katalytischen Effekt auf die Hydrolyse ausüben, so daß diese weitgehend ohne Nebenproduktbildung abläuft. Zweckmäßigerweise wird an dieser Stelle bei einem kontinuierlichen Prozeß die Mutterlauge nach der MethioninFeststoffabtrennung wieder eingesetzt; die Mutterlauge kann dann zusätzlich noch - der Löslichkeit entsprechend - Rest-Methionin enthalten, auch dies hat sich als günstig für das Verfahren herausgestellt.

Die mittlere Verweilzeit der Reaktionslösung in der Hydrolysekolonne beträgt vorteilhaft 10 - 20 Minuten. Das Molverhältnis der Kaliumionenmenge zu der Summe aus 5-(2-Methylmercaptoethyl)-hydantoin + Methionin beträgt günstig bis zu 10, vorzugsweise 1,3 - 5, insbesondere 1,5 - 2. Die erhaltene Ausbeute an Kaliummethioninat in der Reaktionslösung beträgt bei dieser Verfahrensweise typisch 99.0 - 100 %. Die Konzentration an Kaliummethioninat kann durch entsprechende Auswahl der Hydantoinkonzentration oder durch Verdünnen bzw. Einengen der nach der Hydrolyse anfallenden Lösung eingestellt werden.

Gegenstand der vorliegenden Erfindung ist die Freisetzung von Methionin aus Alkalimethioninat, vorteilhaft aus einer wässrigen Lösung, wie sie nach den obigen Verfahrensweisen erhältlich ist. Die Erfindung betrifft ein Verfahren zur Herstellung von Methionin aus Alkalimethioninat in wässriger Lösung durch Freisetzung mit Kohlendioxid, wobei man der wässrigen Lösung, enthaltend das Alkalimethioninat, vor der Freisetzung des Methionins einen Entschäumer zugibt. Die Erfindung betrifft außerdem ein Verfahren zur Herstellung von Methionin aus Alkalimethioninat in wässriger Lösung durch Freisetzung mit Kohlendioxid, wobei man die oben beschriebene Freisetzung in einem Rührzellenreaktor mit intensiver Durchmischung oder in einem Rührreaktor mit quasi idealer Durchmischung durchführt.

Als Entschäumer eignen sich alle Verbindungen, die eine schaumbremsende Funktion ausüben. Vorzugsweise wird der Entschäumer in Form einer Dispersion in die Lösung eingebracht. Hierdurch erhält man eine besonders gute Verteilung in der Lösung und nicht eine Konzentrierung im wesentlichen an der Oberfläche der wässrigen Lösung. Hierdurch wird der günstige Effekt des Entschäumers auf die Freisetzung des Methionins, insbesondere die Vermeidung der Bildung von dünnen Plättchen oder Schuppen, begünstigt. Es entstehen feste kugelige Kristalle, überwiegend mit einem Durchmesser von 100 bis 200 µm. Vorteilhaft wird der Entschäumer in einer Konzentration von 1 000 bis 10 000 ppm, bezogen auf das Gesamtmethionin (Methionin + Methioninat, umgerechnet auf Methionin) zugegeben.

Bei der Freisetzung des Methionins aus der wässrigen Lösung mittels Kohlendioxid ist es besonders vorteilhaft, wenn das Kohlendioxid über eine Düsenvorrichtung im Bereich des Bodens in die wäßrige Lösung eingeleitet wird. Dies begünstigt wiederum die Freisetzung des Methionins. Außerdem führt man die Freisetzung vorteilhaft bei einem Druck von 1 bis 30 bar durch, vorzugsweise auch bei einer Temperatur von 0 bis 100 °C.

Ganz besonders vorteilhaft wird eine wäßrige Lösung eingesetzt, die im wesentlichen frei von Ammoniak ist.

Auch die letzte Verfahrensweise wird besonders günstig kontinuierlich durchgeführt. Vorzugsweise wird die beschriebene Verfahrensweise mit der vorher beschriebenen Verfahrensweise zur Herstellung eines Alkalisalzes von Methionin kombiniert, wobei besonders bevorzugt die gesamte Kombination der oben beschriebenen Verfahrensweisen möglich ist.

Im folgenden wird das Verfahren zur Freisetzung von Methionin anhand des bevorzugten Kalium-D,L-Methioninats beschrieben, wobei auch andere Alkali, z. B. Natrium, möglich sind. Die hierbei angegebenen weiteren bevorzugten oder allgemeinen Verfahrensbedingungen gelten entsprechend auch bei der oben beschriebenen allgemeinen Verfahrensweise.

Bei der Freisetzung von D,L-Methionin aus Kalium-D,L-Methioninat durch Einleiten von Kohlendioxid insbesondere in eine Hydrolyse-Lösung des 5-(2-Methylmercaptoethyl)-hydantoins ist es besonders vorteilhaft, wenn die Lösung praktisch frei von Ammoniak ist. Vorzugsweise enthält die Lösung außerdem gelöstes D,L-Methionin. Außerdem können noch gewisse Mengen an Kaliumcarbonat und Kaliumhydrogencarbonat zugegen sein. Die Lösung kann, sofern gewünscht, vor der Kohlendioxid-Zugabe über Aktivkohle gereinigt werden. Die Kohlendioxid-Zugabe erfolgt üblicherweise bei einer Temperatur von 0 bis 100 °C, vorzugsweise bei 20 bis 35 °C, und üblicherweise bei einem Druck von 1 bis 30 bar, vorzugsweise von 2 bis 5 bar. Vorzugsweise wird das Kohlendioxid so lange in das Reaktionsgemisch eingeleitet, bis ein pH-Wert von ca. 7 bis 9, vorzugsweise 7,5 bis 8,5, erreicht wird und/oder bis die Ausfällung des D,L-Methionins beendet ist. Hierbei ist es besonders vorteilhaft, wenn das Kohlendioxid am Boden eines Reaktors direkt oder zweckmäßigerweise fein verteilt über eine Düsenvorrichtung eingeführt wird. Der Reaktor ist vorteilhaft als Rührzellenreaktor oder als quasi idealer Rührreaktor ausgebildet. Außerdem kann insbesondere bei einer kontinuierlichen Verfahrensweise der Entschäumer zusätzlich noch den Durchsatz erhöhen. Der Entschäumer wird üblicherweise in einer Menge von mindestens 1 000 und zweckmäßig bis 10 000 ppm, vorzugsweise 3 000 bis 5 000 ppm, bezogen auf das in der Reaktionslösung befindliche Gesamtmethionin, insbesondere als wäßrige Emulsion zudosiert. Das freigesetzte Methionin wird vorteilhaft von der Mutterlauge abgetrennt und ist nach dem Trocknen weitgehend staubarm und zeichnet sich durch gute Fließfähigkeit und ein hohes Schüttgewicht aus. Die Methioninpartikel besitzen überwiegend einen Durchmesser von 100 bis 200 µm. Bei dieser Verfahrensweise beträgt die Ausbeute des isolierten D,L-Methionins üblicherweise 98 bis 100 %. Die nach der D,L-Methionin-Abtrennung, insbesondere Filtration, erhaltene Mutterlauge kann vorteilhaft wieder zur Hydrolyse des 5-(2-Methylmercaptoethyl)-hydantoins, ggf. nach Konzentrierung und/oder Abzug von CO₂, eingesetzt werden.

Die Erfindung wird im folgenden anhand von Figuren und Beispielen näher ausgeführt.

Es zeigen
- Fig. 1: eine Verfahrensskizze zur kontinuierlichen Herstellung von 5-(2-Methylmercaptoethyl)-hydantoin;
- Fig. 2: eine Verfahrensskizze zur kontinuierlichen Hydrolyse von 5-(2-Methylmercaptoethyl)-hydantoin zu Alkalimethioninat, und
- Fig. 3: eine Verfahrensskizze zur kontinuierlichen Freisetzung und Isolierung von D,L-Methionin aus Alkali-D,L-Methioninat.

In den Figuren sind der Einfachheit halber Nebenkomponenten, wie Überschuß an NH₃/CO₂, z. B. bei der Ausschleusung, nicht aufgeführt.

Die Beispiele 1 - 6 betreffen die Herstellung des Zwischenprodukts und des Methioninats

### Beispiele zur Herstellung von 5-(2-Methylmercaptoethyl)-hydantoin

Der allgemeine Verfahrensablauf für die Beispiele 1 - 4 ist in Fig. 1 dargestellt. Bei der kontinuierlichen Arbeitsweise wird Cyanwasserstoff über eine Mischdüse 1 mit 3-Methylmercaptopropionaldehyd und einem nachgeschalteten Statikmixer 2 vermischt. In einem Mischreaktor 3 wird eine Lösung von Ammoniak und Kohlendioxid in Wasser hergestellt, wobei diese Komponenten von den nachfolgenden Stufen recycliert werden können. Die beiden Mischungen werden in einen Kreislaufreaktor in das Umsetzungsgemisch kontinuierlich eingespeist. In einer Mischeinrichtung 4 erfolgt eine gute momentane Durchmischung der Lösungen mit dem im Kreislauf geführten Umsetzungsgemisch. Die Umlaufmischung wird über einen Wärmetauscher 5, mit dem die gewünschte Temperatur eingestellt wird, gepumpt 6. Aus dem Kreislauf wird von dem Umsetzungsgemisch kontinuierlich ein entsprechender Anteil abgezogen. Dieser wird dann zur Vervollständigung der Reaktion einem Nachreaktor 7 zugeführt. Das so erhaltene Produktgemisch kann dann ggf. sofort in die nächste Reaktionsstufe überführt werden.

### Beispiel 1:

Es wurde eine Einrichtung wie in Fig. 1 verwendet. Zunächst wurde auf 90 °C angewärmtes Wasser im Kreislauf geführt; dann wurde in den Kreislauf stündlich eine Lösung von 10.5 Mol Cyanwasserstoff in 10 Mol 3-Methylmercaptopropionaldehyd und 6,8 1 einer wässrigen Ammoniumcarbonatlösung, die 9.6 Gew.-% Ammoniak und 15.2 Gew.-% Kohlendioxid enthielt, eingespeist. Die Umwälzung im Kreislauf betrug 300 l pro Stunde. Die Temperatur wurde auf 90 °C gehalten; der Druck betrug 14 bar. Aus dem Kreislauf wurde stetig ein dem Zufluß entsprechendes Volumen des Umsetzungsgemisches abgezogen und zur Nachreaktion geleitet. Die mittlere Verweilzeit war im Kreislauf 10 min und in der Nachreaktion 2 h. Die Ausbeute an 5-(2-Methylmercaptoethyl)-hydantoin, bzw. an auf Methionin verseifbaren Verbindungen, bezogen auf eingesetzten 3-Methylmercaptopropionaldehyd, betrug 99.8 %. (Die Angaben beziehen sich auf 3-Methylmercaptopropionaldehyd als 100 % gerechnet.)

### Beispiel 2:

Es wurde wie in Beispiel 1 verfahren, jedoch wurde zu Beginn statt Wasser eine bei Zimmertemperatur gesättigte Ammoniumcarbonatlösung im Kreislauf geführt, die 9.6 Gew.-% Ammoniak und 15.2 Gew.-% Kohlendioxid enthielt. Die Ausbeute betrug 99.7 %.

### Beispiel 3:

Es wurde wie nach Beispiel 1 verfahren, jedoch wurde die Temperatur im Kreislauf und bei der Nachreaktion auf 115 °C gehalten. Der Druck betrug 16 bar. Die Umwälzung im Kreislauf betrug 150 1 pro Stunde. Die mittlere Verweilzeit war im Kreislauf 6 min und in der Nachreaktion 20 min. Die erreichte Ausbeute betrug 99.9 %.

### Beispiel 4:

Es wurde wie in Beispiel 1 verfahren, jedoch wurden stündlich eine Lösung von 10.1 Mol Cyanwasserstoff in 10.0 mol 3-Methylmercaptopropionaldehyd und 6.8 1 einer wässrigen Ammoniumcarbonatlösung, die 5.5 Gew.-% Ammoniak und 8.5 Gew.-% Kohlendioxid enthielt, eingespeist. Die Temperatur wurde im Kreislauf und bei der Nachreaktion auf 115 °C gehalten. Der Druck betrug 16 bar. Die Durchflußgeschwindigkeit im Kreislauf war 150 l pro Stunde; die mittlere Verweilzeit im Kreislauf war 6 min und in der Nachreaktion 40 min. Die Ausbeute betrug 99.8 %.

### Beispiel 5 und 6

Beispiele zur Herstellung von Kalium-Methioninat-Lösung

Der allgemeine Verfahrensablauf für die Herstellung von Kalium-Methioninat-Lösung ist in Fig. 1 wiedergegeben.

### Beispiel 5:

### Vergleichsbeispiel mit einer Druckapparatur ohne Zirkoneinbauten

Mittels Pumpendruck wird in eine kontinuierlich arbeitende Druckkolonne 8 aus Edelstahl (vgl. Fig. 2), die mit Dampf betrieben wird, stündlich eine Lösung von 100 kg Kaliumhydrogencarbonat in wäßrige Lösung und 41 kg 5-(2-Methylmercaptoethyl)-hydantoin in 400 l Wasser eingespeist. Das Reaktionsgemisch wird dabei auf 180 °C aufgeheizt und hat eine mittlere Verweilzeit von ca. 15 min bei etwa 8 bar. Freigesetztes Ammoniak und Kohlendioxid wird am Kopf der Reaktorkolonne über ein Druckhalteventil abgezogen. Am Boden des Druckreaktors wird die Reaktionslösung entspannt und mit dem Wärmetauscher 9 abgekühlt. Dabei werden stündlich 41.9 kg Kalium-Methioninat in Lösung gewonnen (94.5 % d. Th.).

### Beispiel 6:

Es wird eine Apparatur gemäß Fig. 2 verwendet, die eine Druckkolonne beinhaltet, welche Einbauten aus Zirkon besitzt.

Mit einer Pumpe werden stündlich 553 kg 5-(2-Methylmercaptoethyl)-hydantoin in 1600 l Reaktionslösung aus der Hydantoinherstellung nach Abb. 1 und 3550 l eines Gemisches aus Kaliumcarbonat/Kaliumhydrogencarbonat und Kaliumhydroxid in wässriger Lösung aus der Mutterlaugenrückführung nach der Methioninfeststoffabtrennung mit einem Kaliumgehalt von 140 g pro Liter und einem Rest-Methioningehalt von 120 g pro Liter auf den Kopf einer Druckhydrolysekolonne 8 mit Zirkoneinbauten eingespeist. Die Reaktionstemperatur beträgt 165 °C; der Reaktionsdruck beträgt 7 bar. Das freiwerdende Ammoniak und Kohlendioxid werden am Kopf der Kolonne über ein Druckhalteventil ausgeschleust und der 5-(2-Methylmercaptoethyl)-hydantoin-Synthese erneut zugeführt.

Am Sumpf der Druckapparatur werden 5150 l pro Stunde eines wässrigen Gemisches mit 96.5 g Kalium pro Liter und 175 g Methionin pro Liter (entsprechend einer Zunahme von 476 kg/h Methionin im Gesamtsystem) erhalten (Ausbeute: 100 %). Die Reaktionslösung wird über einen Wärmetauscher 9 abgekühlt und der Methionin-Freisetzung zugeführt.

### Beispiel 7

### Beispiel zur Freisetzung von D,L-Methionin aus Kalium-D,L-methioninat (Fig. 3)

Am Kopf eines Rührreaktors 10 von 340 1 Fassungsvermögen werden kontinuierlich 686 1 pro Stunde einer wässrigen Lösung mit 83,6 kg Kalium-D,L-methioninat (Hydrolyselösung von 5-(2-Methylmercaptoethyl)-hydantoin mit zusätzlich 39,77 kg Kreislauf-Methionin und Kaliumverbindungen eingespeist. Gleichzeitig wird am Reaktorboden Kohlendioxid zugeführt, so daß im Reaktor ein Druck von 2 - 3 bar herrscht. Ebenso werden 0,38 kg pro Stunde Entschäumer in Form einer wässrigen Emulsion in den Reaktor eindosiert; diese Menge entspricht ca. 3940 ppm Entschäumer pro Kilogramm Gesamt-Methionin. Die Reaktionstemperatur wird bei 25 °C gehalten. Um im Reaktor ein konstantes Niveau zu halten, werden am unteren Teil des Reaktors der Zudosierung entsprechende Mengen an Reaktionslösung ausgeschleust. Die Ausschleussuspension wird abfiltriert, wobei stündlich 66,5 kg Fest-D,L-Methionin (als Trockensubstanz gerechnet) erhalten werden und die Mutterlauge mit einem Restgehalt von 39,77 kg D,L-Methionin als Verseifungsagens in die Hydantoin-Hydrolysestufe rezykliert werden kann. Die Ausbeute ist quantitativ.

## Patentansprüche

1. Verfahren zur Herstellung von DL-Methionin aus einer wässrigen Alkalimethioninatlösung durch Freisetzung mit Kohlendioxid,
**dadurch gekennzeichnet,**
**daß** man der das Alkalimethioninat enthaltenden wässrigen Lösung, vor der Freisetzung des Methionins einen Entschäumer zugibt.

2. Verfahren nach Anspruch 1,
**dadurch gekennzeichnet,**
**daß** man die Freisetzung in einem Rührzellenreaktor mit intensiver Durchmischung oder in einem Rührreaktor mit quasi idealer Durchmischung durchführt.

3. Verfahren nach den der Ansprüchen 1, oder 2,
**dadurch gekennzeichnet,**
**daß** man den Entschäumer in Form einer Dispersion zuführt.

4. Verfahren nach einem der Ansprüche 1, 2 oder 3,
**dadurch gekennzeichnet,**
**daß** man den Entschäumer in einer Konzentration von 1 000 bis 10 000 ppm bezogen auf Gew.-Teile Gesamtmethionin zugibt.

5. Verfahren nach einem der Ansprüche 1 bis 4,
**dadurch gekennzeichnet,**
**daß** man das Kohlendioxid über eine Düsenvorrichtung im Bereich des Bodens in die wäßrige Lösung einleitet.

6. Verfahren nach einem der Ansprüche 1 bis 5,
**dadurch gekennzeichnet,**
**daß** man die Freisetzung bei einem Druck von 1 bis 30 bar durchführt.

7. Verfahren nach einem der Ansprüche 1 bis 6,
**dadurch gekennzeichnet,**
**daß** man die Freisetzung bei einer Temperatur von 0 bis 100 °C durchführt.

8. Verfahren nach einem der Ansprüche 1 bis 7,
**dadurch gekennzeichnet,**
**daß** man eine wässrige Alkalimethioninatlösung einsetzt, erhältlich durch Hydrolyse von 5-(2-Methylmercaptoethyl)-hydantoin in Gegenwart einer wässrigen Lösung enthaltend Alkali und Kohlendioxid.

9. Verfahren gemäß Anspruch 8, **dadurch gekennzeichnet, dass** die Hydrolyse zumindest zu Beginn in Gegenwart von mind. 0,1 eq, insbesondere bis 7 eq, Ammoniak je Equivalent 5-(2-Methylmercaptoethyl)-hydantoin durchgeführt wird.

10. Verfahren gemäß den Ansprüchen 8 oder 9, dass die Hydrolyse in Gegenwart von metallischem Zirkonium oder ener Zirkonium-Legierung, enthaltend mind. 10 Gew.-% Zirkonium, durchgeführt wird.

11. Verfahren nach Anspruch 10, **dadurch gekennzeichnet, dass** die Hydrolyse zumindest zu Beginn in Gegenwart von mind. 0,1 eq, insbesondere bis 7 eq, Ammoniak je Equivalent 5-(2-Methylmercaptoethyl)-hydantoin durchgeführt wird.

12. Verfahren nach einem der Ansprüche 8 bis 11, **dadurch gekennzeichnet, dass** man die Alkalikomponente equimolar oder im Überschuß, bezogen auf das 5-(2-Methylmercaptoethyl)-hydantoin einsetzt.

13. Verfahren nach einem der Ansprüche 1 bis 8, **dadurch gekennzeichnet, dass** man eine wässrige Kaliummethioninatlösung einsetzt.

14. Verfahren nach einem der Ansprüche 1 bis 8, **dadurch gekennzeichnet, dass** die wässrige Lösung im Wesentlichen frei von Ammoniak ist.

15. Verfahren nach einem der Ansprüche 1 bis 14, **dadurch gekennzeichnet, dass** es kontinuierlich durchgeführt wird.

16. Verfahren nach einem der Ansprüche 1 bis 15, **dadurch gekennzeichnet, dass** die Methioninfreisetzung in den gesamt-kontinuierlich verlaufenden Prozess mit den Verfahrensstufen 5-(2-Methylmercaptoethyl)-hydantoinbildung, Hydantoinhydrolyse und Methioninatbildung und Methioninfreisetzung integriert ist.
